(19) Europäisches Patentamt
European Patent Office
Office européen des brevets

(11) **EP 0 965 349 A1**

(12) **EUROPÄISCHE PATENTANMELDUNG**

(43) Veröffentlichungstag:
22.12.1999 Patentblatt 1999/51

(51) Int. Cl.⁶: **A61K 38/18**, A61K 33/06,
A61K 33/42
// (A61K38/18, 33:06, 33:42)

(21) Anmeldenummer: 98250222.1

(22) Anmeldetag: 18.06.1998

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(71) Anmelder: Roche Diagnostics GmbH
68298 Mannheim (DE)

(72) Erfinder:
• Lehmann, Paul
67549 Worms (DE)

• Gottschalk, René
65929 Frankfurt/Main (DE)

(74) Vertreter:
Ziebig, Marlene, Dr. Dipl.-Chem. et al
Patentanwälte
Gulde Hengelhaupt Ziebig
Lützowplatz 11-13
10785 Berlin (DE)

(54) **Verwendung von Erythropoietin zur Behandlung von Hämochromatosen**

(57) Die vorliegende Erfindung betrifft die Verwendung von Epo zur Herstellung von pharmazeutischen Präparaten zur Behandlung von Hämochromatosen sowie pharmazeutische Kombinationspräparate enthaltend Erythropoietin (Epo) and Calcium- und/oder Phosphatverbindungen. Diese Kombinationspräparate werden insbesondere zur Behandlung von primärer Hämochromatose eingesetzt.

EP 0 965 349 A1

**Beschreibung**

Die vorliegende Erfindung betrifft die Verwendung von Erythropoietin (Epo) in niedriger Dosierung zur Herstellung von pharmazeutischen Präparaten zur Behandlung von Hämochromatosen sowie pharmazeutische Kombinationspräparate enthaltend Erythropoietin (Epo) and Calcium- und/oder Phosphatverbindungen. Diese Kombinationspräparate werden insbesondere zur Behandlung primärer Hämochromatosen (ererbten Eisenüberladungsstörungen) eingesetzt.

Mehr als 2/3 (ca. 70%) der neugebildeten Knochenmarkstammzellen werden im Eisen- und Knochenstoffwechsel des Organismus zu Erythrozyten und Osteoblasten/Osteoklasten funktioniert. Diese Vorgänge (Erythropoese und Knochenbildung) werden durch die beiden Differenzierungshormone Erythropoietin (Epo) and Parathyroidhormon (PTH) gesteuert, wobei eine normale PTH-Konzentrationen im Serum (zwischen 10 bis 60 ng/l) zu normaler Osteoblasten/Osteoklasten-Bildung führt und normale Epo-Konzentrationen, die zwischen 6-25 U/l liegen, eine normale Erythrozytenbildang bewirken.

Lebenswichtige Bedeutung kommt insbesondere der Erythropoese zu, denn 45% der neugebildeten Stammzellen des Knochenmarks werden zu Erythrozyten entwickelt. Aus diesem Grund sind alle Parameter (Eisenresorption, Eiseneinbau, Epo, Folat, Vitamin B12) des Eisenstoffwechsels extrem eng reguliert.

Bekanntermaßen ist Epo ein in der Niere gebildetes und auch synthetisch herstellbares Glykoprotein, das auf dem Blutweg („humoral") die Blutbildung (Erythropoese) anregt.

Es ist bekannt, die Anämie, insbesondere die transfusionsbedingte Anämie vom Hämodialysepatienten (renale Anämien), mit rekombinantem humanem Erythropoietin (rhEpo) zu therapieren. Die Anämie bei chronischen Krankheiten ist weltweit die zweithäufigste Anämieform.

Im Vordergrund der Anämien, die durch verminderte Erythropoese im Knochen oder durch Störungen in der Eisenreutilisation verursacht werden, steht eine verminderte Erythrozytenneuproduktion. Der tägliche Eisenbedarf für eine normale Erythropoese beträgt 25 mg. Eisenmangelanämien, die die häufigste Form der hypochromen Anämie sind, werden durch Zufuhr von Eisen reguliert. Eine kombinierte Gabe mit rhEpo wird therapeutisch genutzt, um einen signifikanten Anstieg der Erythrozytenzahl zu bewirken.

In der klinischen Chemie wird für die Diagnose von Eisenstoffwechselstörungen die Serumferritin-Konzentration bestimmt. Das Gesamtkörpereisen beträgt bei Männeren ca. 3,5 g, bei Frauen 2,5 g. Eisen befindet sich im aktiven Metabolismus und in Speicherkompartimenten. Im aktiven Pool befinden sich bei einem Mann durchschnittlich 2100 mg im Hämoglobin, 200 mg im Myoglobin, 150 mg in Enzymen der Gewebe (Häm und Nicht-Häm) und 3 mg im Eisentransport-Kompartiment. Eisen wird im Gewebe intrazellulär als Ferritin (700 mg) und als Hämosiderin (300 mg) gespeichert.

Der genaue Resorptionsmechanismus für Eisen ist noch nicht vollständig geklärt (Gunshin et al., Nature 388, 482-488, 1997), führt wahrscheinlich über das Protein DCT1. Die Regulation erfolgt in entscheidender Weise durch die Dünndarm-Mukosezellen. Das entscheidende Signal für die Mukosa scheint der Gesamteisengehalt des Körpers zu sein. Es wurde gezeigt, daß die Serum-Ferritinkonzentration invers korreliert mit der Menge des aufgenommenen Eisens.

Das Eisen wird von den intestinalen Mukosezellen an das Transferrin übergeben. Dieses Eisentransportprotein hat zwei Eisenbindungsstellen. Es wird in der Leber synthetisiert. Damit existiert ein Mechanismus, durch den Eisen von Zellen (z.B. Dünndarmmukosa, Makrophagen) übernommen und an spezifische Membranrezeptoren von Erythroblasten, Plazentazellen oder Leberzellen abgegeben werden kann. Der Transferrin-Eisen-Rezeptor-Komplex gelangt durch Endozytose in die Erythrozyten-Vorläuferzellen, wo das Eisen an die Mitochondrien weitergegeben wird. Dort wird aus Eisen and Protoporphyrin Häm gebildet.

Nicht für die Erythropoese benötigtes Eisen wird mittels Transferrin in zwei Arten des Speicherpools verlagert. Der wichtigste Speicher ist das Ferritin. Hierbei handelt es sich um eine heterogene Familie von Proteinen, die einen Eisenkern umschließen. Es ist löslich und stellt die aktive Speicherform in Leber (Hepazyten), Knochenmark, Milz (Makrophagen), Erythrozyten und im Serum (ca. 100 ng/ml) dar. Der Gewebeferritinpool ist sehr labil und rasch verfügbar, wenn Eisen benötigt wird. Das zirkulierende Serum-Ferritin stammt aus dem retikuloendothelialen System (RES), and seine zirkulierende Konzentration geht parallel mit dem Gesamtkörpereisen (jedes ng/ml entspricht 8 mg Eisenvorrat).

Zusammenfassend ist festzustellen, daß im Eisenstoffwechsel

-   alle Zellen des RES Speicher für Ferritin und Hämosiderin sind,
-   Hb-synthetisierende, Eisen-verbrauchende Stammzellen durch Epo zu Erythrozyten entwickelt werden und
-   der Transport des Eisens aus dem Speicher zum entstehenden Erythrozyten und umgekehrt aus dem abgestorbenen Erythrozyten in den Speicher durch Transferrin erfolgt.

In Knochenstoffwechsel fungiert in Analogie zum Eisenstoffwechsel

- der Knochen als Speicher für Hydroxylapatit-Kristalle (and Kollagen), der Osteoklast „labilisiert" den Hydroxylapatit, indem er ihn bis zu einem Grundgerüst abbaut. Liegt keine Störung vor, wird das Grundgerüst zur Synthese verwendet.
- Hydroxylapatit (und Kollagen) synthetisierende, Calcium und Phosphat verbrauchende Stammzellen werden durch PTH (+IGF 1+2) zu Osteoblasten entwickelt und
- der Transport des Calciums und Phosphates erfolgt aus dem Speicher zum entstehenden Osteoblasten und umgekehrt nach der Synthese des Hydroxylapatits in den Speicher.

Beschrieben wurde bereits 1983 durch S.G. Massry (Kidney International 24, (16) 204-207) und auch durch F.N. Hutchison und J. Jones (AJKD, Vol. 29, No. 5, Mai 1997), daß bei Vorliegen eines Hyperparathyroidismus, also bei sehr hohen PTH-Konzentrationen die Erythropoese unterdrückt ist. Das heißt durch eine hohe PTH-Konzentration (>> 60 ng/l) wird demzufolge die Epo-Bildung im Organismus gedrosselt, wodurch zwangsweise auch die Bildung von Erythrozyten erniedrigt wird.

PTH ist demzufolge ein wichtiger Regulator im menschlichen Organismus, denn sowohl Eisen- wie auch Knochenmetabolismen werden durch PTH reguliert. PTH regelt naturgemäß die Resorption von Calcium, Phosphat, Magnesium und Eisen.

Es können aber folgende Schwachstellen in Eisen- und Knochenstoffwechsels auftreten:

- ungenügende Differeuzierung der Stammzellen durch Epo bzw. PTH,
- „Labilisierung" der Speicherform (Eisenkern in Ferritin bzw. des Hydroxylapatits in Collagen durch Osteoklast),
- ungenügender Transport von Eisen mm Erythrozyten bzw. Hydroxylapatit (Calcium und Phosphat) zu den Osteoblasten.

Während man bei Störungen der Erythropoese im Falle renaler Anämien und Eisenmangelanämien bereits Möglichkeiten der Therapie kennt, sind Hämochromatosen therapeutisch nicht gelöst. Hier gibt es bis heute nur die Möglichkeit des Aderlasses.

Hämochromatosen sind Eisenspeicherkrankheiten infolge Eisenüberladung des Organismus, vor allem der parenchymatösen Organe, die mit einer erhöhten Eisenabsorption and massiver Ablagerung des Eisens in Form des Hämosiderins in zahlreichen Organen sowie im Monozyten-Makrophagen-System einhergehen. Unterschieden werden primäre (idiopathische) Hämochromatosen und sekundäre (erythropoetische) Hämochromatosen. Sekundäre Hämochromatosen sind Blutbildungsstörungen, die zu Hämosiderose (erworbene Eisenüberladung des Organismus) führen. Die primären (idiopathischen) Hämochromatosen manifestieren sich mit Leberzirrhose (Pigmentzirrhose), bronzeartiger Hautverfärbung und Ausfallserscheinungen endo- und exokriner Drüsen (Hypogonadismus, insulinabhängiger Diabetes mellitus = „Bronzediabetes"), Herzinsuffienz, Haarverlust.

Der Erfindung lag deshalb die Aufgabe zugrunde, pharmazeutische Präparate bereitzustellen, die zur Behandlung von Hämochromatosen geeignet sind und optimal abgestimmte Mengen an Wirkstoffen enthalten.

Es wurde überraschend gefunden, daß Erythropoietin-Präparate in geringer Dosierung zur Behandlung von Hämochromatosen geeignet sind.

Die Erfindung betrifft deshalb die Verwendung von Epo zur Herstellung pharmazeutischer Präparate zur Behandlung von Hämochromatosen.

Erfindungsgemäß weisen die Präparate 500 bis 5.000 U Epo auf und werden vorzugsweise zur Behandlung von Patienten mit sekundären Hämochromatosen eingesetzt.

Als geeignete Erythropoietin-Präparate im Sinne der vorliegenden Erfindung kommen solche Wirkstoffe in Frage, die hinsichtlich der physiologischen Wirkung des humanen Erythropoietins vergleichbar sind. Für derartige Wirkstoffe wird im Sinne der vorliegenden Erfindung kurz die Bezeichnung Epo bzw. Epo-Präparat verwendet. Geeignete Epo-Präparate sind beispielsweise das rekombinante Epo (rhEpo; vgl. Europäische Patentschriften EP-B1 0 205 564 bzw. EP-B1 0 411 678) oder auch entsprechende Modifikationen derartiger Proteine. Als Modifikationen kommen beispielsweise solche Proteine mit höherem (vgl. EP 0148 605) oder geringerem Molekulargewicht als 34.000 Da (SDS-PAGE-Molekulargewicht des urinären Epo) in Frage, ebenso Isoformen des Enzyms oder Proteine mit unterschiedlicher Glykosilierung. Insbesondere können auch durch PEG (Polyethylenglykol) chemisch modifizierte Proteine verwendet werden. Ferner kommen grundsätzlich auch solche Proteine in Frage, die sich durch Deletionen, Substitutionen oder Verlängerungen von einzelnen oder mehreren Aminosäuren von der Aminosäuresequenz des naturlichen Epo mit einer Länge von 166 Aminosäuren ableiten. Derartige Proteine besitzen im wesentlichen vergleichbare physiologische Eigenschaften wie rhEpo. Insbesondere weisen derartige Proteine biologische Eigenschaften auf, daß Knochenmarkszellen veranlaßt werden, die Produktion von Retikulozyten und roten Blutkörperchen zu steigern und/oder die Hämoglobinsynthese zu steigern. Anstelle derartiger Proteine können auch niedermolekulare Substanzen verwendet werden, die als Epo-Mimetika bezeichnet werden, and die an den gleichen biologischen Rezeptor binden. Diese Mimetika können vorzugsweise auch oral verabreicht werden. Die in verabreichende Menge derartiger Proteine oder Mimetika wird ermittelt durch Vergleich der biologischen Aktivitäten zwischen Epo und diesen Wirkstoffen.

Für die Behandlung beinhaltet das erfindungsgemäße Kombinationspräparat beispielsweise 500 bis 5000 U (anstelle der Abkürzung „U" kann auch die Abkürzung „JE" für Internationale Einheiten verwendet werden) eines Epo-Präparates. Bevorzugte Dosierungen sind 500 U, 1.000 U, 2.000 U and 5.000 U pro Einzeldosierung.

Es wurde weiterhin gefunden, daß die Eisenresorption jedoch quantitativ verglichen mit einer Calcium(phosphat)-Resorptionsstörung eine ganz geringe Rolle spielt (1:1000 ist das Verhältnis der Konzentrationen) und daß bei der primären Hämochromatose neben einer Eisenresorptionsstörung vor allem eine Calcium(phosphat)-Resorptionsstörung vorliegt.

So wurde festgestellt, daß zum einen die Erythropoese nur bedient wird, wenn Transferrin voll beladen werden kann (Tf x 2), wenn die Eisenspeicher also voll sind. Umgekehrt werden die Speicher mit halbbeladenem Transferrin aufgefüllt (Tf x 1).

Zum anderen wird der Osteoblast nur mit genügend Calcium and Phosphat versorgt, wenn der Speicher (Knochen) voll Hydroxylapaptit ist. Ist das nicht der Fall, hat das dann beim Eisen fatale Folgen. Es wird zu viel Eisen resorbiert, weil zu viel Calcium(phosphat) resorbiert wird. Da Epo durch PTH gedrosselt wird, wird die Erythropoese nicht bedient.

Das kann erfindungsgemäß durch gezielte Gabe von Epo in niedriger Dosierung in Kombination mit Calcium- und/oder Phosphatverbindungen vermieden werden.

Gegenstand der Erfindung ist deshalb auch die Verwendung von Epo in geringer Dosierung und Calcium und/oder Phosphatverbindungen zur Herstellung eines pharmazeutischen Präparates zur Behandlung primärer Hämochromatosen sowie ein Kombinationspräparat aus einem Erythropoietin-Präparat und Calcium- und/oder Phosphatverbindungen, das für die Therapie von primärer Hämochromatose geeignet ist. Die Wirkstoffe des Kombinationspräparates können sowohl in getrennten Darreichungsformen als auch in einer Darreichungsform vorliegen.

Das Kombinationspräparat enthält eine für die Therapie einer primären Hämochromatose optimal abgestimmte Menge an Epo and Calcium und/oder Phosphat. Neben Epo enthält es erfindungsgemäß bevorzugt eine Calciumphosphatkomplexverbindung. Ganz besonders bevorzugt enthält es eine Apatitverbindung der Formel $Ca^{2+}[Ca_3(PO_4)_2]_3{}^{2-}$, die vorzugsweise in Form eines Glukonates vorliegen kann. Das Kombinationspräparat kann aber auch als Calciumverbindung Calcium-Glukonat und/oder als Phosphatverbindung Glukose-1-Phosphat oder Kaliumhydrogenphosphat enthalten.

Das bevorzugte Verhältnis von Calcium zu Phosphat (bzw. Phosphor) beträgt im Kombinationspräparat von 11:5 bis 9:7, vorzugsweise 10:6.

Das Kombinationspräparat kann auch Epo nur in Kombination mit einer Calciumverbindung enthalten oder nur in Kombination mit einer Phosphatverbindung

In einer bevorzugten Variante enthält das Kombinationspräparat 500-5000 U rhEPO, 20-500 mg Calcium- und/oder 10-250 mg Phosphor (bzw. 30-750 mg Phosphat). Diese Werte sind so zu verstehen, daß eine entsprechende Calcium- oder Phosphatverbindung in einer solchen Menge zu applizieren ist, daß 20 bis 500 mg Calcium bzw. 10 bis 250 mg Phosphor (bzw. 30-750 mg Phosphat) zugeführt werden.

Die Kombinationspräparate der vorliegenden Erfindung können Epo and die Calcium- und/oder Phosphatverbindungen in einer verkaufsfertigen Verpackungseinheit nebeneinander konfektioniert enthalten (sogenannte Kombinationsverpackung). Die Arzneimittelpackungen können außerdem entweder eine geeignete Menge an Epo oder eine geeignete Menge einer Calciumphosphatverbindung in Form eines Einzelpräparates enthalten, wobei die Einzelpräparate hinsichtlich der Menge der Inhaltsstoffe derart konfektioniert sind, daß sie im Sinne der Erfindung für die kombinierte Gabe mit dem jeweils anderen Präparat verabreicht werden können. In diesen Fällen werden vom Hersteller oder dem Arzneimittel-Importeur den Präparaten in der Regel ein in vielen Ländern gesetzlich vorgeschriebener Beipackzettel für Arzneimittel beigelegt, in dem Anweisungen oder Informationen über die kombinierte Gabe der Einzelpräparate enthalten sind. Dies trifft auch auf Arzneimittelpackungen zu, die eine geeignete Menge an Epo und eine geeignete Menge einer Calciumverbindung oder einer Phosphatverbindung enthalten bzw. einer Calciumverbindung und einer Phosphatverbindung.

Im Sinne der Erfindung kommen als Calcium-/Phosphat-Präparate orale oder parenterale Darreichungsformen in Frage. Es kann sich grundsätzlich um Einzelpräparate handeln, die als Wirkstoff ein physiologisch verträgliches Calciumsalz und/oder eine Phosphatverbindung oder eine Calcium-Phosphatkomplex-Verbindung enthalten, oder auch um Kombinationspräparate, die neben dem physiologisch verträglichen Präparaten weitere Wirkstoffe, wie z.B. Vitamine, Folsäure, Thiaminchiorid, Riboflavin, Pyridoxin, Ascorbinsäure, Nicotinamid, etc. enthalten.

Im Sinne der vorliegenden Erfindung ist es auch möglich, Epo and Calcium- und/oder Phosphatverbindungen in Form von getrennten pharmazeutischen Formulierungen (freie Kombination) gleichzeitig oder aber auch nacheinander zu applizieren. Diese freie Kombination, die in einer Verpackungseinheit zur Verfügung gestellt werden kann, hat den Vorteil der großen Flexibilität.

In der Regel wird die freie Kombination in Form einer einzigen Verpackungseinheit zur Verfügung gestellt, die mindestens zwei Behältnisse umfaßt, wobei das erste eine geeignete Darreichungsform für Epo-Präparat ist (Lyophilisat, Injektions- oder Infusionslösung, ggf auch zur oralen Applikation), und das zweite eine geeignete Darreichungsform für das Calciumphosphat- oder Calcium- und/oder Phosphat-Präparat darsteilt. Dadurch kann jedem Patienten individuell eine direkt zuzuordnende Menge an Epo und Calcium-/Phosphatverbindung zur Verfügung gestellt werden. Derartige Kombinationspräparate bieten darüber hinaus den Vorteil des größeren Therapieerfolges, da jeweils die optimal abgestimmte Menge der Einzelpräparate festgelegt ist, und deren Verwechslung mit sonst im Handel erhältlichen Einzelpräparaten, die in untersehiedlichen Dosierungen angeboten werden, weitgehend ausgeschlossen werden kann.

Die erfindungsgemäßen Kombinationspräparate minimieren ferner das Risiko einer versehentlich zu hohen Calcium- und/oder Phosphatgabe, die möglicherweise erfolgen kann, wenn herkömmliche Calcium- oder Phosphatpräparate aus separaten Arzneimittelpackungen zusammen mit Epo appliziert werden. Durch die erfindungsgemäßen Kombinationationspräparate wird eine sichere Therapie und einfache Handhabung sichergestellt. Im vorliegenden Fall ist es auch möglich, einen Wirkstoff als Injektionslösung und den anderen Wirkstoff als Darreichungsform zur oralen Verabreichung einzusetzen.

Für den Fall, daß das Epo-Präparat als Lyophilisat zur Verfügung gestellt wird, enthalten die Arzneimittelpakkungen (Kombinationspackungen) die entsprechende Menge des Epo-Präparates in Glasampullen oder in Karpulen. Das Calcium-/Phosphat-Präparat kann in fester Form (Tablette, Pulver, Granulat, Lyophilisat, etc.) oder auch in flüssiger Form in getrennten Behältnissen vorliegen. Ferner enthält die Kombinationspackung eine Rekonstitutionslösung, um entweder das Epo-Lyophilisat allein oder auch zusammen mit dem festen Calcium-/Phosphat-Präparat aufzulösen. Liegt das Calcium/Phosphat-Präparat als gebrauchsfertige Lösung vor, kann die Lösung zusammen mit der Epo-Lösung gemischt werden, falls die gemeinsame Applikation erfolgen soll. Grundsätzlich kann das Calcium-/Phosphat-Phäparat auch als Konzentrat für den Zusatz zu herkömmlichen Infusionslösungen zur Verfügung gestellt werden, wodurch eine langsamere Applikation über mehrere Stunden hinweg erfolgen kann.

Eine weitere Möglichkeit im Sinne der Erfindung besteht darin, jeweils einzelne, verkaufsfertige Darreichungsformen für Epo-Präparate und Calcium-und/oder Phosphatverbindungen als unabhängige Arzneimittel zur Verfügung zu stellen, wobei die Einzelpräparate derart konfektioniert sind, daß sie die erforderlichen Mengen an Einzelsubstanzen für die erfindungsgemäße Kombination des Epo-Präparates und Calcium und/oder Phosphat enthalten.

Bei der Anwendung der Kombinationspräparate ist es auch möglich, Epo und Calcium- und/oder Phosphatverbindungen in einer sogenannten fixen Kombination, d.h., in einer einzigen pharmazeutischen Formulierung zu verabreichen, in der beide Verbindungen enthalten sind. Dies kann z.B. eine Injektionslösung- bzw. Infusionslösung oder deren Lyophilisat sein, die beispielsweise in Ampullen abgefüllt sind. Die fixe Kombination der jeweiligen Wirkstoffe in Form eines Lyophilisates hat den Vorteil der einfachen und sicheren Handhabung. Das Lyophilisat wird in der Ampulle durch Zugabe pharmazeutisch üblicher Injektionsmedien gelöst und intravenös appliziert.

Die Herstellung der pharmazeutischen Darreichungsformen erfolgt nach üblichen, in der galenisehen Technik bekannten Verfahren mit pharmazeutisch üblichen Hilfsstoffen.

Bei der Durchführung der Kombinationstherapie mit dem erfindungsgemäßen Kombinationspräparat müssen

a) die Ferritin-Konzentration als Maß für Füllungszustand des Eisenspeichers,
b) Transferrinsättigung und Transferrinrezeptor für die Beladungskapazität des Transportproteins sowie mittels verschiedener diagnostischer Parameter
c) der Füllzustand des Knochens mit Apatit und mit Kollagen festgestellt werden.

Die Zielwerte, die in der Regel auf eine gute Einstellung des Eisen- und Knochenstoffwechsels hinweisen, können den Tabellen 1 und 2 entnommen werden:

Tabelle 1

| Diagnostik | |
|---|---|
| Ferritin (ng/ml) | ≥ 100 |
| | Warngrenze: 400 |
| Transferrinsättigung (%) | ≥ 15 |
| löslicher Transferrin-Rezeptor (mg/l) | ≤ 2 |

Tabelle 2

| Analyt | Serum | | Produkt Ca x $PO_4$ in Serum [mg/dl]$^2$ | Urin [mmol/l] |
|---|---|---|---|---|
| | [mmol/l] | [mg/dl] | | |
| Ca | 2,5 | 10 | | < 5 |
| | | | 40 | |
| Phosphat | 1 | 4 | | < 20 |
| AP, bone | < 150 U/l | | | |
| Osteocalcin | 5 - 100 µl/l | | | |
| <u>Crosslinks</u> | <u>im Urin</u> | | | |
| Pyrodinolin | <100µmol/mol Creatinin | | | |
| 3-Hydroxy-pyrodinolin | < 20µmol/mol Creatinin | | | |
| NTx =N-terminal crosslinked peptide | methodenabhängig | | | |
| PTH | 10-70 ng/l | | | |

Bei der Durchführung der Therapie sind also deshalb im wesentlichen die folgenden diagnostischen Parameter zu kontrollieren:

a) für Eisenstoffwechsel;

- Ferritin (ng/ml)
- Transferrinsättigung(%)/Transferrinrezeptor (mg/l)
- die Syntheseleistung der Stammzellen anhand von hämatologischen Kontrollparametern wie Hämoglobin (Hb), Hämatokrit (Anteil der roten Blutkörperchen am Gesamtvolumen), Erythrozyten, hypochrome Erythrozyten, Retikulozyten, hypochrome Retikulozyten.

Ferritin ist ein Eisen speicherndes Glykoprotein, von dem gewebetypische Isoferritine existieren und das im Serum immunologisch bestimmt werden kann. Der Ferritin-Wert ist ein Maß für die Eisenspeicherung im Gewebe. In den meisten Laboratorien liegt der Normalbereich zwischen 30 und 300 ng/ml, und der geometrische Mittelwert beträgt 88 bei Männern und 49 bei Frauen. Die Serum-Ferritin-Werte stehen in enger Beziehung zum Eisengesamtvorrat des Körpers. Deshalb findet man erhöhte Spiegel bei Eisenüberladung.

Zur Ermittlung des Ferritin-Spiegels werden in heparinisiertem (bzw. EDTA, Citrat) Blut Erythrozyten von den Leukozyten and Thrombozyten (die ebenfalls Ferritin enthalten) mittels Zentrifugation getrennt. Es folgen die Lyse der Erythrozyten und die immunologische Bestimmung des gespeicherten Ferritins. Das Erythrozyten-Ferritin spiegelt den Status der Eisenspeicher während der zurückliegenden 3 Monate wider (d.h. während der Lebenszeit eines Erythrozyten). Die Normalwerte liegen im allgemeinen zwischen 5 und 48 Attogramm (ag) pro Erythrozyt. Werte < 5 findet man bei Eisenmangelanämien, erhöhte Werte (oft > 100) bei Eisenüberladung.

Häufig wird auch die Methode der Bestimmung des Serum-Ferritins angewendet. So kann z.B. der automatisierbare Ferritin-Test von Boehringer Mannheim eingesetzt werden, der auf dem Prinzip des immunologischen Agglutinationstests mit Reaktionsverstärkung durch Latex beruht.

Die Transferrinsättigung ist definiert als das Verhältnis von Serum/Plasma-Eisenkonzentration zu Serum/Plasma-Transferrin-Konzentration (multipliziert mit einem Korrekturfaktor von 1,41). Es handelt sich hierbei um eine dimensionslose Zahl, die unabängig vom Status des Patienten ist. Sie berechnet sich nach der Formel:

$$\text{Transferrinsättigung}(\%) = (\text{Eisen[mg/dl]} \times 100)/(\text{Transferrin[mg/dl]} \times 1,41)$$

Auch der Serumtransferrin-Rezeptor kann durch enzymverstärkten Immunabsorptions-test (enzyme-linked immunosorbent assay = ELISA) oder Latex-Test bestimmt werden. Dabei wird ein monoklonaler Antikörper gegen den löslichen Rezeptor verwendet. Der Referenzbereich liegt zwischen 0,5-3 mg/l.

b) Diagnostische Parameter für den Knochenstoffwechsel sind:

- Ca x PO$_4$ im Serum (in mg/dl)$^2$, sowie Ca und Phosphat im Serum und im Urin (in mmol/l),
- der Füllungszustand des Apatits im Kollagen, über den Nachweis von bone AP, Pyridinoline („Crosslinks" und „Crosslabs"), 3-Hydroxyprolin und NTx,
- die Konzentration des Osteocalcins, das auch Apatit speichern und transportieren kann. Es kommt im Serum in einer dem Ferritin vergleichbaren Konzentration vor (ca. 10 - 100 μg/l).

Die Normalwerte für Calcium und Phosphat liegen im Serum bei 2,2-2.6 mmol/l, das entspricht 8,6-10,2 mg/dl, (Calcium) und bei 1,0-1,5 mmol/l, das entspricht 2,7-4,5 mg/dl, (Phosphat). Daraus resultiert ein Produkt für Ca x PO$_4$, das bei Werten zwischen 23(mg/dl)$^2$ (8,6 x 2,7) und 46 (mg/dl)$^2$ (10,2 x 4,5), als akzeptierbar angesehen werden kann.
Betrachtet man die Zusammensetzung des im Knochen vorkommenden Hydroxylapatits (Ca$_{10}$(PO$_4$)$_6$(OH)$_2$) so ist bei einem Produkt Ca x PO$_4$ von ca. 40 (mg/dl)$^2$ eine optimale Beladungskapazität gegeben (vgl. Tabelle 3).

Tabelle 3

|  | mmol/l |  | mmol/l |
|---|---|---|---|
| Ca$_{Serum}$ | 2,2-2,6 | Phosphat$_{Serum}$ | 1,0-1,5 |
| Ca$_{Apatit}$ | 2,2-2,6 | Phosphat$_{Apatit}$ | 1,3-1,6 |

Der Nachweis des Kollagens, das das wichtigste Speicherprotein des Apatits ist, erfolgt indirekt über Marker des Knochenaufbaus im Serum, wie z.B. bone ALP und terminale Propeptide, die bei der Bildung von Kollagenfibrillen abgespalten werden, sowie über Marker des Knochenabbaus, wie Pyrodinoline (Crosslinks), 3-Hydroxy-Prolin oder NTx (N-terminale crosslinked peptide) im Urin.
Auch das Matrixprotein Osteocalcin, dessen Glutaminsäurereste es befähigen an die im Knochen eingelagerten Kristalle des Calcium-Minerals Hydroxyapatit zu binden, kommt im Serum in einer Konzentration von ca. 10 - 100 μg/l vor (Normalwert).
Das Produkt Calcium x Phosphat stellt für die diagnostischen Untersuchungen die wichtigste Größe dar und wird im Serum bestimmt. Es wurde gefunden, daß der Knochen ausreichend mit Ca und PO$_4$ gefüllt ist, wenn das Produkt Ca x PO$_4$ 30-50 (mg/dl)$^2$ beträgt, wobei der Zielwert 40 (mg/dl)$^2$ sein sollte. Als Anzeichen einer Organverkalkung ist ein Wert > 60 (mg/dl)$^2$ Ca x PO$_4$ anzusehen. Bei Werten < 25 mg/dl drohen Hypocalcämie bzw. Hypophosphatämie, die dann zur erhöhten PTH-Ausschüttung führen und die Drosselung von Epo bewirken können.
Die vorliegende Erfindung betrifft demzufolge die Verwendung von Epo in niedriger Dosierung zur Herstellung pharmazeutischer Präparate zur Behandlung von Hämochromatosen. Zur Behandlung sekundärer Hämochromatosen weisen die Präparate 500 - 5.000 U Epo auf. Die Behandlung erfolgt ein- bis fünfmal, bevorzugt bis zu viermal wöchentlich, wobei die Gesamtmenge an Epo von 5.000 U pro Patient und Woche nicht überschritten wird.
Weiterhin betrifft die Erfindung die Verwendung von Epo in Kombination mit Calcium und/oder Phosphatverbindungen zur Herstellung von Kombinationspräparaten zur Behandlung von primären Hämochromatosen sowie pharmazeutische Kombinationspräparate umfassend Erythropoietin (Epo) and Calcium- und/oder Phosphatverbindungen, wobei die Verbindungen in getrennten Darreichungsformen oder in einer einheitlichen Darreichungsform vorliegen können. Insbesondere kommen folgende Ausführungsformen in Frage:
Kombinationspräparate, die Epo and eine Calciumphosphatkomplexverbindung enthalten. Kombinationspräparate, die als Calciumphosphatkomplexverbindung eine Apatitverbindung der Formel Ca$^{2+}$[Ca$_3$(PO$_4$)$_2$]$_3$$^{2-}$ enthalten, vorzugsweise in Form eines Glukonates. Kombinationspräparate, die Epo, eine Calcium- und eine Phosphatverbindung enthalten. Kombinationspräparate, die als Phosphatverbindung Glukose-1-Phosphat oder Kaliumhydrogenphosphat enthalten. Kombinationspräparate, wobei das Verhältnis Calcium zu Phosphat zwischen 11:5 bis 9:7 liegt, und vorzugsweise 10:6 beträgt. Kombinationspräparate, die 500-5000 U Epo, 20-500 mg Calcium und/oder 10-250 mg Phophor (bzw. 30-750 mg Phosphat) in getrennten Darreichungsformen als Injektions- bzw. Infusionslösungen oder als Lyophilisate oder in einer einheitlichen Darreichungsform aufweisen.
Bei der Durchführung der Kombinationstherapie kann mit dem erfindangsgemäßen Kombinationspräparat auf einfache Art and Weise über die wöchentliche maximale Dosierung entschieden werden. Die Behandlung erfolgt ein- bis fünfmal, bevorzugt bis zu viermal wöchentlich, wobei die Gesamtmenge an Epo von 5.000 U und an Calcium von 4 x 500 mg pro Patient and Woche nicht überschritten wird.
Um eine Überproduktion an Erythrozyten zu vermeiden, kann es während der jeweiligen Behandlungen von Vorteil sein, parallel eine Aderlaßtherapie durchzuführen.

Die vorliegende Erfindung betrifft ferner Verfahren zur Herstellung von pharmazeutischen Epo-Präparaten und Kombinationspräparaten wie oben angegeben, wobei man Epo, Calcium- und/oder Phosphatverbindungen mit pharmazeutisch üblichen Träger- oder Hilfsstoffen formuliert und in einer einheitlichen oder getrennten Darreichungsform zur Verfügung stellt.

Die Erfindung bezieht sich ferner auf die Verwendung von EPO zur Herstellung eines pharmazeutischen Präparates zur Behandlung sekundärer Hämochromatosen sowie die Verwendung von Epo und Calcium- und/oder Phosphatverbindungen zur Herstellung von Kombinationspräparaten zur Behandlung primärer Hämochromatosen.

Anschließend wird die Erfindung an Beispielen näher erläutert.

Beispiel 1:

Patienten mit primärer Hämochromatose, deren Ferritin-Wert oberhalb von 400 ng/ml liegt und deren Calcium x Phosphat-Wert unterhalb von $(20 \text{ mg/dl})^2$ liegt, werden dreimal pro Woche mit 1.000 U rhEpo behandelt. Außerdem erhalten die Patienten dreimal pro Woche 500 mg Calcium und 300 mg Phosphor, vorzugsweise in Form einer $Ca[Ca_3(PO_4)_2]_3$-Glukonat-Lösung, die oral gegeben wird. Die beiden Präparate werden den Patienten jeweils am gleichen Tag verabreicht. Durch Bestimmung der diagnostischen Parameter, insbesondere der Transferrinsättigung wird der Eisenstatus der Patienten erfaßt. Diese Behandlung wird solange fortgeführt, bis der Ferritin- und der Calcium x Phosphat-Wert im Normalbereich liegen.

Beipiel 2

Patienten mit sekundärer Hämochromatose, deren Ferritin-Wert oberhalb von 400 ng/ml liegt, werden fünfmal pro Woche mit 500 U rhEpo behandelt. Durch Bestimmung der Transferrinsättigung wird der Eisenstatus der Patienten erfaßt. Diese Behandlung wird solange fortgeführt, bis der Ferritin-Wert im Normalbereich liegt.

**Patentansprüche**

1.  Verwendung von Erythropoietin (Epo) in niedriger Dosierung zur Herstellung eines pharmazeutischen Präparates zur Behandlung von Hämochomatosen.

2.  Verwendung nach Anspruch 1, dadurch gekennzeichnet, daß Epo im pharmazeutischen Präparat in Dosierungen von 500-5000 U vorliegt.

3.  Verwendung nach Anspruch 1, dadurch gekennzeichnet, daß das Epo-enthaltende Präparat zur Behandlung sekundärer Hämochromatosen eingesetzt wird.

4.  Verwendung nach Anspruch 1 oder 2, dadurch gekennzeichnet, daß Epo in Kombination mit Calcium- und/oder Phosphatverbindungen zur Herstellung eines pharmazeutischen Präparates zur Behandlung primärer Hämochromatosen eingesetzt wird.

5.  Verwendung nach Anspruch 4, dadurch gekennzeichnet, daß Epo im pharmazeutischen Präparat in Dosierungen von 500-5000 U vorliegt sowie 20-500 mg Calcium und/oder 10-250 mg Phosphor enthalten sind.

6.  Verwendung nach Anspruch 4 oder 5, dadurch gekennzeichnet, daß das Calcium/Phosphat-Präparat eine Calciumphosphatkomplexverbindung enthält, vorzugsweise eine Apatitverbindung der Formel $Ca^{2+}[Ca_3(PO_4)_2]_3^{2-}$.

7.  Verwendung nach Anspruch 6, dadurch gekennzeichnet, daß die Apatitverbindung in Form eines Glukonates vorliegt und Calcium:Phospat im Verhältnis 11:5 bis 9:7, vorzugsweise 10:6 enthalten sind.

8.  Pharmazeutisches Kombinationspräparat umfassend Erythropoietin (Epo) und Calcium- und/oder Phosphatverbindungen, wobei die Verbindungen in getrennten Darreichungsformen oder in einer einheitlichen Darreichungsform vorliegen können.

9.  Kombinationspräparat nach Anspruch 8, dadurch gekennzeichnet, daß es Epo und eine Calciumphosphatkomplexverbindung enthält.

10. Kombinationspräparat nach Anspruch 9, dadurch gekennzeichnet, daß die Calciumphosphatkomplexverbindung eine Apatitverbindung der Formel $Ca^{2+}[Ca_3(PO_4)_2]_3^{2-}$ ist, vorzugsweise in Form eines Glukonates.

**11.** Kombinationspräparat nach Anspruch 8, dadurch gekennzeichnet, daß es Epo, eine Calcium- und eine Phosphatverbindung enthält.

**12.** Kombinationspräparat nach einem der Anspüche 8 bis 11, dadurch gekennzeichnet, daß das Verhältnis Calcium zu Phosphat zwischen 11:5 bis 9:7, vorzugsweise 10:6 beträgt.

**13.** Kombinationspräparat nach einem der Ansprüche 8 bis 12, dadurch gekennzeichnet, daß es 500-5.000 U Epo, 20-500 mg Calcium- und/oder 10-250 mg Phosphor enthält.

**14.** Pharmazeutische Verpackungseinheit umfassend 500-5.000 U Epo und 20-500 mg Calcium und/oder 10-250 mg Phosphor in getrennten Darreichungsformen als Injektions- bzw. Infusionslösungen oder als Lyophilisate.

Europäisches
Patentamt

**EUROPÄISCHER RECHERCHENBERICHT**

Nummer der Anmeldung

EP 98 25 0222

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EP 0 269 394 A (KIRIN AMGEN INC) 1. Juni 1988 * siehe insbesondere Ansprüche 1 & 3; Seite 3, Zeilen 10-25; und Tabelle 1* | 1-3 | A61K38/18 A61K33/06 A61K33/42 //(A61K38/18, 33:06,33:42) |
| X | WO 97 48411 A (HIGUCHI MASATO ;KATO JUNJI (JP); NIITSU YOSHIRO (JP); CHUGAI PHARM) 24. Dezember 1997 *siehe insbesondere Zusammenfassung* | 1-3 | |
| X | US 4 992 419 A (WOOG HEINRICH ET AL) 12. Februar 1991 * siehe die Beispiele * | 8,11,13 | |
| A | JONES-LECOINTE A ET AL: "Sub-optimal doses of human recombinant erythropoietin markedly lower serum ferritin." CLINICAL AND LABORATORY HAEMATOLOGY, (1991) 13 (3) 251-3. JOURNAL CODE: DKF. ISSN: 0141-9854., XP002084484 ENGLAND: United Kingdom * siehe insbesondere Zusammenfassung, sowie Seite 252, 2. Zeile * | 1-3 | |
| | | | **RECHERCHIERTE SACHGEBIETE (Int.Cl.6)** A61K |
| A | URENA P ET AL: "Serum erythropoietin and erythropoiesis in primary and secondary hyperparathyroidism: effect of parathyroidectomy." NEPHRON, (1991) 59 (3) 384-93. JOURNAL CODE: NW8. ISSN: 0028-2766., XP002084485 Switzerland * siehe insbesondere die Zusammenfassung * | 4-14 | |

Der vorliegende Recherchenbericht wurde für alle Patentansprüche erstellt

| Recherchenort | Abschlußdatum der Recherche | Prüfer |
|---|---|---|
| MÜNCHEN | 16. November 1998 | Isert, B |

**ANHANG ZUM EUROPÄISCHEN RECHERCHENBERICHT
ÜBER DIE EUROPÄISCHE PATENTANMELDUNG NR.**

EP 98 25 0222

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-11-1998

| Im Recherchenbericht angeführtes Patentdokument | | Datum der Veröffentlichung | Mitglied(er) der Patentfamilie | | Datum der Veröffentlichung |
|---|---|---|---|---|---|
| EP 0269394 | A | 01-06-1988 | US 5013718 | A | 07-05-1991 |
| | | | AU 602028 | B | 27-09-1990 |
| | | | CA 1322165 | A | 14-09-1993 |
| | | | DE 3773852 | A | 21-11-1991 |
| | | | DK 407488 | A | 21-07-1988 |
| | | | GR 3003254 | T | 17-02-1993 |
| | | | IE 60865 | B | 24-08-1994 |
| | | | JP 6092316 | B | 16-11-1994 |
| | | | JP 63159322 | A | 02-07-1988 |
| | | | KR 9509100 | B | 14-08-1995 |
| | | | PT 86187 | B | 07-11-1990 |
| | | | WO 8803808 | A | 02-06-1988 |
| WO 9748411 | A | 24-12-1997 | AU 3107197 | A | 07-01-1998 |
| | | | JP 10067678 | A | 10-03-1998 |
| US 4992419 | A | 12-02-1991 | DE 3729863 | A | 16-03-1989 |
| | | | AU 2173988 | A | 27-04-1989 |
| | | | CA 1330301 | A | 21-06-1994 |
| | | | CN 1031801 | A,B | 22-03-1989 |
| | | | CS 8805901 | A | 13-12-1990 |
| | | | DD 273004 | A | 01-11-1989 |
| | | | DE 3872334 | A | 30-07-1992 |
| | | | DK 483188 | A | 06-03-1989 |
| | | | EP 0306824 | A | 15-03-1989 |
| | | | ES 2051806 | T | 01-07-1994 |
| | | | FI 884051 | A,B, | 06-03-1989 |
| | | | GR 3005454 | T | 24-05-1993 |
| | | | HK 89495 | A | 16-06-1995 |
| | | | IE 60310 | B | 29-06-1994 |
| | | | JP 1071818 | A | 16-03-1989 |
| | | | JP 2057196 | C | 23-05-1996 |
| | | | JP 7080782 | B | 30-08-1995 |
| | | | KR 9609929 | B | 25-07-1996 |
| | | | LV 10178 | A,B | 20-10-1994 |
| | | | LV 10393 | A,B | 20-02-1995 |
| | | | MX 12880 | A | 01-12-1993 |
| | | | NO 178687 | B | 05-02-1996 |
| | | | PH 25618 | A | 08-08-1991 |
| | | | PT 88417 | A,B | 31-07-1989 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT
## ÜBER DIE EUROPÄISCHE PATENTANMELDUNG NR.

EP 98 25 0222

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-11-1998

| Im Recherchenbericht angeführtes Patentdokument | Datum der Veröffentlichung | Mitglied(er) der Patentfamilie | Datum der Veröffentlichung |
|---|---|---|---|
| US 4992419 A | | RU 2043118 C | 10-09-1995 |
| | | RU 2100032 C | 27-12-1997 |

EPO FORM P0461

Für nähere Einzelheiten zu diesem Anhang : siehe Amtsblatt des Europäischen Patentamts, Nr. 12/82